# EUROPÄISCHE PATENTSCHRIFT

(11) **EP 3 496 657 B1**
(45) Veröffentlichungstag und Bekanntmachung des Hinweises auf die Patenterteilung: **07.10.2020**
(21) Anmeldenummer: 17755112.4
(22) Anmeldetag: 09.08.2017
(51) Int. Cl.: A61K 6/818

(54) **ROHLING UND VERFAHREN ZUR HERSTELLUNG EINES ZAHNERSATZTEILS**
BLANK AND METHOD FOR PRODUCING A TOOTH REPLACEMENT PART
ÉBAUCHE ET PROCÉDÉ DE FABRICATION D'UN ÉLÉMENT PROTHÉTIQUE DENTAIRE

(30) Priorität: 09.08.2016 DE 102016214725
(43) Veröffentlichungstag der Anmeldung: 19.06.2019
(73) Patentinhaber: Sirona Dental Systems GmbH, 64625 Bensheim (DE)
(72) Erfinder: BÄURER, Michael, 75015 Bretten (DE)
(74) Vertreter: Özer, Alpdeniz
(86) Internationale Anmeldenummer: PCT/EP2017/070172
(87) Internationale Veröffentlichungsnummer: WO 2018/029244

(56) Entgegenhaltungen:
- EP-A1- 2 000 109
- EP-A1- 2 939 993
- EP-A2- 1 859 757
- DE-A1-102013 226 497
- DE-A1-102016 202 902

## Beschreibung

### Technisches Gebiet

Die Erfindung betrifft ein Verfahren und einen Rohling zur Herstellung eines Zahnersatzteils unter Verwendung einer CAD/CAM-Vorrichtung aufweisend einen Block aus einem Sintermaterial.

### Stand der Technik

Aus dem Stand der Technik sind mehrere Rohlinge zur Herstellung von Zahnersatzteilen unter Verwendung einer CAD/CAM-Vorrichtung bekannt.

EP 2000109 A1 offenbart ein Verfahren zur Herstellung eines Dentalteils aus einem vorgesinterten Rohling, der aus einem Keramikmaterial hergestellt wurde, wobei mittels einer maschinellen Bearbeitung des Rohlings und das anschließende Sintern des Rohlings auch seine endgültige Dichte in einem Sinternvorgang bei einer Temperatur von 1300° bis 1650° das Dentalteil hergestellt wird. Der Rohling kann dabei bei einer Temperatur zwischen 800 °C bis 1100 °C vor gesintert werden.

EP 2939993 A1 offenbart einen pinkfarbenen einen Zirkoniumoxid-Sinterkörpers mit einer hohen Dichte und Festigkeit des gesinterten Körpers und einer für ein Dentalmaterial, insbesondere für orthodontische Brackets, nützlichen farbigen Transluzenz. Der gefärbte, lichtdurchlässige Zirkoniumoxid-Sinterkörper enthält dabei in eine Ausführungsform Yttriumoxid, Erbiumoxid und Eisenoxid.

DE 10 2013 226 497 A1 offenbart ein Verfahren zur Planung einer Sinterung eines Zahnersatzteils, wobei in Abhängigkeit von den Geometrieparametern des Zahnersatzteils ein Temperaturprofil bestimmt wird. Die Heizrate kann dabei zwischen 100°C/Minute und 400°C/Minute aufweisen.

EP1859757 A2 offenbart Zr02 Rohlinge, und ein Verfahren zu deren Herstellung, bei dem a) nanoskaliges Zr02-Pulver (mit einer spezifischen Oberfläche von mindestens 100 m2 /g) mit einer farbgebenden Substanz beschichtet wird (z.B. Tb203), b) die beschichteten Pulver vorzugsweise klassiert und mindestens ein gefärbtes Pulver in eine Pressform gefüllt wird, c) das oder die gefärbten Pulver zu einem Formkörper gepresst werden und d) der verpresste Formkörper zu einem Rohling bei einer Temperatur von 800 bis 1300°C vorgesintert wird, sowie die Verwendung dieser Rohlinge zur Herstellung von dentalen Restaurationen.

Ein voreingefärbter Rohling wird oft aus einem Zirkoniumdioxid (ZrO₂) hergestellt. Als Farbstoff wird oft Eisenoxid (Fe₂O₃) zur Gelbfärbung verwendet. Als Zusatz können die Rohlinge auch Aluminiumoxid (Al₂O₃) enthalten. Die Rohlinge werden vor der Bearbeitung in der CAM-Vorrichtung in einem Sinterofen bei einer Temperatur von bis zu 1150 °C vorgesintert. Die Rohlinge bestehen meistens aus einem Pulver aus keramischen Partikeln, die mittels eines Polymers zusammengehalten werden. Das Polymer kann dabei etwa einen Gewichtsanteil von 3 % aufweisen. Beim Vorsintern im Sinterofen wird das Polymer ausgebrannt und die Keramikpartikel angesintert, sodass der Rohling fest genug ist, um mittels der CAM-Bearbeitungsmaschine bearbeitet zu werden. Das Zahnersatzteil wird dann aus dem vorgesinterten Rohling mittels der CAD/CAM-Vorrichtung herausgearbeitet. Das hergestellte Zahnersatzteil wird anschließend im Sinterofen dichtgesintert, wobei die notwendige Dichte und Festigkeit des Zahnersatzteils erreicht wird.

Ein Nachteil dieser Rohlinge besteht darin, dass beim Dichtsintern größerer Zahnersatzteile, wie Brücken, in einer Aufheizphase eine Heizrate höchstens 50°C/ Minute betragen darf. Denn ein schnelleres Aufheizen mit einer höheren Heizrate würde zu mechanischen Spannungen innerhalb des Zahnersatzteils und zu einem möglichen Bruch führen. Außerdem bleibt der Kern eines größeren Zahnersatzteils bei zu schnellem Sintern porös, wobei der Rand des Zahnersatzteils schneller durchsintert.

Die Aufgabe der vorliegenden Erfindung besteht daher darin, einen Rohling bereitzustellen, der in der Aufheizphase höhere Heizraten erlaubt, ohne dass ein Bruch des Zahnersatzteils entsteht und ohne dass ein unerwünschter ästhetischer Eindruck des Zahnersatzteils durch einen porösen Kern entsteht.

### Darstellung der Erfindung

Der Gegenstand der Erfindung ist in den Ansprüchen 1 und 6 offenbart.

Die Erfindung betrifft einen Rohling zur Herstellung eines Zahnersatzteils unter Verwendung einer CAD/CAM-Vorrichtung aufweisend einen Block aus einem Sintermaterial. Der Block aus dem Sintermaterial ist bei einer Ansintertemperatur zwischen 1000 °C und 1250 °C in einen Sinterofen bereits vorgesintert.

Das Sintermaterial ist dabei ein Pulver aus Keramikpartikeln mit einem Zirkoniumdioxid (Y-ZrO₂) - Gewichtsanteil von mindestens 90 %, wobei eine spezifische Oberfläche des Pulvers zwischen 11 m²/g und 17 m²/g beträgt.

Der Rohling kann beispielsweise eine Rohlingshalterung und den Block aufweisen, wobei der Rohling in den Abmessungen des Blocks den herkömmlichen Rohlingen für bekannte CAM-Bearbeitungsmaschinen entsprechen kann. Das herzustellende Zahnersatzteil kann beispielsweise eine Vollprothese für ein Implantat, eine Dentalprothese, eine Vollkrone, eine Teilkrone, eine Brücke aus mehreren ganzen Zähnen oder ein Inlay sein. Die CAD/CAM-Vorrichtung umfasst eine CAD-Einheit, wie ein Computer, zur Planung und grafischen Anzeige eines 3D-Modells des herzustellenden Zahnersatzteils und eine CAM-Bearbeitungsmaschine zum Herausarbeiten des herzustellenden Zahnersatzteils aus einem eingespannten Rohling nach dem geplanten 3D-Modell. Das Sintermaterial des Blocks kann aus einem Pulver keramischer Partikel, beispielsweise aus Zirkoniumdioxid, bestehen.

Als Bindemittel kann ein Polymer oder eine Polymermischung enthalten sein. Als Bindemittel kann beispielsweise ein zweiphasiges Bindersystem aus einer Polymermischung verwendet werden. Der Block aus dem Sintermaterial ist bereits vorgesintert. Beim Vorsintern wird der Rohling bei der Ansintertemperatur zwischen 1000 °C und 1250 °C für eine Dauer von beispielsweise zwei Stunden vorgesintert. Dabei wird der Rohling langsam mit einer Heizrate von höchstens 5 °C pro Minute bis zu der Ansintertemperatur aufgeheizt. Die Ansintertemperatur wird dann für eine Haltezeit von höchstens zwei Stunden gehalten, wobei anschließend der vorgesinterte Rohling abgekühlt wird. Die Heizrate von 5 °C pro Minute darf nicht überschritten werden, da dies zu mechanischen Verspannungen und Brüchen innerhalb des Rohlings führen würde. Nach dem Vorsintern ist der Rohling fest genug, um mittels der CAM-Bearbeitungsmaschine bearbeitet zu werden. Der Sinterofen kann ein herkömmlicher Sinterofen sein, der durch thermische Heizelemente aufgeheizt wird. Der Sinterofen kann aber auch ein Induktionsofen sein, wobei eine Ofenkammer durch Induktion erhitzt wird. Der Sinterofen kann auch ein Entbinderungsofen (engl. Bisque firing furnace) sein.

Die spezifische Oberfläche des Pulvers, aus keramischen Partikeln, zwischen 11 m²/g und 17 m²/g ermöglicht, dass das Zahnersatzteil schneller durchgesintert wird, wobei der gewünschte ästhetische Eindruck des Zahnersatzteils erreicht wird. Der gewünschte ästhetische Eindruck entsteht dadurch, dass keine Restporen im Kern des Zahnersatzteils entstehen, die zu unerwünschtem Streulicht im Inneren des Zahnersatzteils führen.

Zirkoniumdioxid ist ein klassisches Sintermaterial und ist besonders gut zur Herstellung von Zahnersatzteilen geeignet.

Ein Vorteil dieses Rohlings besteht darin, dass durch die höhere Ansintertemperatur als bei herkömmlichen Rohlingen eine höhere Festigkeit und Wärmeleitfähigkeit des vorgesinterten Rohlings erreicht wird. Dadurch wird in einer Aufheizphase beim Dichtsintern des Zahnersatzteils eine höhere Heizrate, wie beispielsweise 100 °C pro Minute, ermöglicht, ohne dass das Zahnersatzteil bricht. Die Ansintertemperatur darf jedoch nicht zu hoch sein. Denn eine zu hohe Festigkeit des vorgesinterten Rohlings würde zu einem höheren Verschleiß der Werkzeuge der CAM-Bearbeitungsmaschine führen. Durch die höhere Heizrate wird also die Dauer des Dichtsinterns des Zahnersatzteils verkürzt.

Vorteilhafterweise kann die Ansintertemperatur zwischen 1050 °C und 1200 °C betragen.

Bei einer Ansintertemperatur zwischen 1050 °C und 1200 °C, wie beispielsweise 1150 °C, wird eine ideale Festigkeit des vorgesinterten Rohlings erreicht.

Vorteilhafterweise kann der Block mit einem Pressdruck zwischen 130 und 250 MPa gepresst sein.

Die Blöcke werden mittels einer Presse aus dem Sintermaterial, bestehend aus keramischen Partikeln und einem Polymer, mit dem Pressdruck zwischen 130 und 250 MPa gepresst. Nach dem Vorsintern kann die resultierende Dichte des Blocks 3,4 g/cm³ bei einer Ansintertemperatur von 1050 °C und 3,6 g/cm³ bei einer Ansintertemperatur von 1100 °C betragen.

Vorteilhafterweise kann die spezifische Oberfläche des Pulvers zwischen 12 m²/g und 14 m²/g betragen.

Bei einer solchen spezifischen Oberfläche des Pulvers werden besonders hohe Heizraten beim Dichtsintern erreicht. Vorteilhafterweise kann das Sintermaterial des Blocks einen Yttriumoxid-Gewichtsanteil zwischen 2 % und 4,5 % enthalten.

Yttriumoxid ist ein Keramikvorstoff und wird als ein klassischer Stabilisator von Zirkoniumdioxid verwendet. Falls der Yttriumoxid-Gewichtsanteil zu gering ist, kann dies zu einem Bruch des Zahnersatzteils in der Abkühlphase führen. Das Sintermaterial des Blocks enthält mindestens einen Farbstoff, eine Kombination mehrerer Farbstoffe und/oder mindestens ein Oxid oder ein Chlorid eines Farbstoffs zur Färbung des Blocks, wobei der mindestens eine Farbstoff Terbium für eine Gelbfärbung ist, der keinen Wertigkeitswechsel bei schnellem Abkühlen aufweist und damit keine Farbveränderung durchläuft.

Die Farbstoffe sind auch Magnesium, Kobalt, Mangan und/ oder Erbium.

Der Farbstoff Erbium ist für eine Rotfärbung als Farbstoff des Blocks geeignet, da dieser Farbstoff, wie Terbium, keinen Wertigkeitswechsel bei schnellem Abkühlen aufweist und damit keine Farbveränderung durchläuft. Im Vergleich dazu weist der Farbstoff Eisenoxid zur Gelbfärbung einen Wertigkeitswechsel bei zu schnellem Abkühlen auf, so dass das Zahnersatzteil beim Abkühlen eine unerwünschte Grünfärbung erhält. Dies führt zu unerwünschten Farbgebungen des Zahnersatzteils. Die Grünfärbung wird dadurch verursacht, dass Sauerstoff nicht schnell genug wieder in die Kristallgitter der Keramik eingebaut werden kann. Als Farbstoffe können auch Oxide oder Chloride der oben genannten Farbstoffe verwendet werden.

Die Erfindung betrifft weiterhin ein Verfahren zur Herstellung eines Zahnersatzteils aus einem Rohling aufweisend einen Block aus einem Sintermaterial. Der Block aus dem Sintermaterial wird bei einer Ansintertemperatur zwischen 1000 °C und 1250 °C in einen Sinterofen vorgesintert.

Bei diesem Verfahren wird der o. g. Rohling verwendet. Durch diese Ansintertemperatur wird die erforderliche Festigkeit des vorgesinterten Rohlings zur Bearbeitung in der CAM-Bearbeitungsmaschine erreicht.

Ein Vorteil dieses Verfahrens besteht darin, dass durch die höhere Ansintertemperatur als bei bekannten vorgesinterten Rohlingen eine höhere Festigkeit und damit höhere Heizraten beim Dichtsintern des hergestellten Zahnersatzteils ermöglicht werden.

Vorteilhafterweise kann der Block aus dem Sintermaterial bei der Ansintertemperatur zwischen 1050 °C und 1200 °C vorgesintert werden.

Bei einer solchen Ansintertemperatur wird die gewünschte Festigkeit des vorgesinterten Rohlings erreicht. Vorteilhafterweise kann der Block mittels einer Pressvorrichtung mit einem Pressdruck zwischen 130 und 250 MPa gepresst werden.

Bei einem solchen Pressdruck der Pressvorrichtung wird die gewünschte Dichte des gepressten Rohlings erreicht. Vorteilhafterweise kann das Zahnersatzteil unter Verwendung einer CAD/CAM-Vorrichtung aus dem vorgesinterten Rohling herausgearbeitet werden.

Die Werkzeuge der CAM-Bearbeitungsmaschine der CAD/CAM-Vorrichtung sind dabei geeignet, um den vorgesinterten Rohling zu bearbeiten.

Das herausgearbeitete Zahnersatzteil wird in einem Sinterofen nach einem festgelegten Temperaturprofil dichtgesintert. Der Sinterofen wird nach einem festgelegten Temperaturprofil gesteuert, sodass ein gewünschter Verlauf der Temperatur innerhalb der Ofenkammer erreicht wird. Das Temperaturprofil kann beispielsweise aus einer Datenbank unterschiedlicher Temperaturprofile ausgewählt werden oder individuell in Abhängigkeit von den Abmessungen des hergestellten Zahnersatzteils, die aus dem geplanten 3D-Modell des Zahnersatzteils bekannt sind, berechnet werden. Das Dichtsintern kann eine erste Trocknungsphase mit einer ersten Heizrate, eine Aufheizphase mit einer zweiten Heizrate, eine Haltephase mit einer festgelegten Haltetemperatur für eine festgelegte Haltezeit und eine Abkühlphase mit einer festgelegten Abkühlrate umfassen.

Bei einem computergestützten Verfahren kann innerhalb des Volumens des geplanten Zahnersatzteils mittels eines Suchalgorithmus eine virtuelle größtmögliche Kugel ermittelt werden. Der Durchmesser einer solchen größtmöglichen Kugel im Volumen des geplanten Zahnersatzteils dient dann als ein Geometrieparameter bei der Auswahl oder Bestimmung eines passenden Temperaturverlaufs für das Dichtsintern.

Das Zahnersatzteil weist eine größtmögliche Kugel im Volumen des Zahnersatzteils mit einem Durchmesser unterhalb eines Grenzwertes von 6 mm auf, wobei das Temperaturprofil in einer Aufheizphase eine festgelegte Heizrate zwischen 150 °C/Minute und 350 °C/Minute aufweist.

Vorteilhafterweise kann das Zahnersatzteil eine größtmögliche Kugel im Volumen des Zahnersatzteils mit einem Durchmesser unterhalb eines Grenzwertes von 3 mm aufweisen, wobei die festgelegte Heizrate zwischen 200 °C/Minute und 350 °C/Minute beträgt.

Beim Durchmesser der größtmöglichen Kugel von kleiner als 3 mm kann die Heizrate beispielsweise 250 °C/ Minute betragen.

Beispielweise kann das Zahnersatzteil eine größtmögliche Kugel im Volumen des Zahnersatzteils mit einem Durchmesser oberhalb eines Grenzwertes von 6 mm aufweisen, wobei das Temperaturprofil in einer Aufheizphase eine festgelegte Heizrate zwischen 70 °C/Minute und 150 °C/Minute aufweist. Beim Durchmesser der größtmöglichen Kugel oberhalb von 6 mm dürfen die Heizrate und eine Abkühlrate beispielsweise beim Zirkoniumdioxid 150 °C/ Minute nicht überschreiten. Denn eine höhere Heizrate könnte zu thermischen Spannungen beim Sintern und dadurch zu Rissen im Zahnersatzteil führen. Darüber hinaus könnten unerwünschte Poren im Kern des Zahnersatzteils entstehen, die die gewünschten ästhetischen Transluzenzeigenschafen stören würden.

### Kurzbeschreibung der Zeichnungen

Die Erfindung wird anhand der Zeichnungen erläutert. Es zeigt, die
- Fig. 1: eine Skizze zur Verdeutlichung des vorliegenden Verfahrens zur Herstellung eines Zahnersatzteils, die
- Fig. 2: ein Temperaturprofil für das Vorsintern, die
- Fig. 3: ein Temperaturprofil 40 für das Dichtsintern des hergestellten Zahnersatzteils. Ausführungsbeispiele

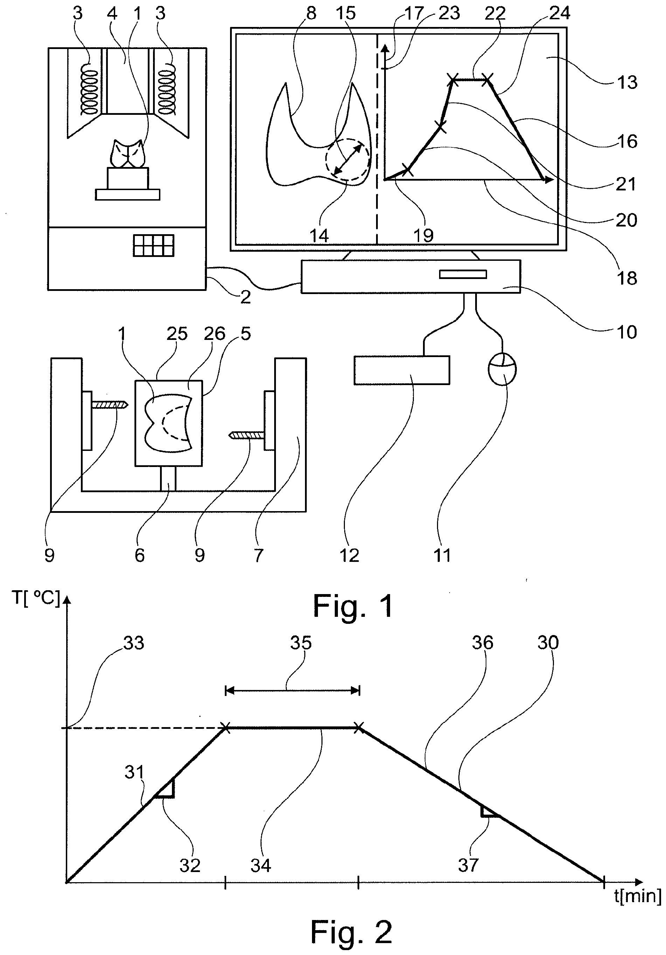

Die Fig. 1 zeigt eine Skizze zur Verdeutlichung des vorliegenden Verfahrens zur Herstellung eines Zahnersatzteils 1 mittels eines Sinterofens 2. Der Sinterofen weist dabei Heizelemente 3 auf, um die Ofentemperatur innerhalb einer Ofenkammer 4 zu regulieren. Alternativ kann auch ein Induktionsofen verwendet werden, der die Ofentemperatur innerhalb der Ofenkammer 4 reguliert. Im ersten Schritt wird ein Rohling 5 mittels eines Sinterofens bei einer Ansintertemperatur zwischen 1000 °C und 1250 °C vorgesintert. Als Sinterofen wird üblicherweise ein großer Industrieofen verwendet, der gleichzeitig mehrere Rohlinge vorsintern kann. Anschließend wird der vorgesinterte Rohling mit einer Rohlingshalterung 6 in eine CAM-Bearbeitungsmaschine 7 eingespannt.

Das herzustellende Zahnersatzteil 1 wird nach einem geplanten 3D-Modell 8 des Zahnersatzteils 1 mittels der CAM-Bearbeitungsmaschine mit Bearbeitungswerkzeugen 9 herausgearbeitet. Die Planung des 3D-Modells erfolgt mittels eines Computers 10, an den Bedienungselementen, wie eine Maus 11 und eine Tastatur 12, angeschlossen sind. Das Anzeigen des 3D-Modells 8 erfolgt mittels einer Anzeigevorrichtung 13, wie eines Monitors. Mittels eines Computerverfahrens wird eine größtmögliche virtuelle Kugel 14 innerhalb des Volumens des 3D-Modells 8 gesucht. Ein Durchmesser 15 der größtmöglichen virtuellen Kugel 14 ist dabei ein wesentliches Maß zur Bestimmung eines Temperaturprofils 16. Die Temperatur 17 ist dabei in Abhängigkeit von der Zeit 18 aufgetragen. Das Temperaturprofil 16 für das Dichtsintern des hergestellten Zahnersatzteils 1 weist typischerweise eine Trocknungsphase 19 mit einer ersten Heizrate, eine erste Aufheizphase 20 mit einer zweiten Heizrate, eine zweite Aufheizphase 21, mit einer dritten Heizrate, eine Haltephase 22 bei einer festgelegten Haltetemperatur 23 und eine Abkühlphase 24 mit einer festgelegten Abkühlrate auf. Ein passendes Temperaturprofil 16 wird beispielsweise aus einer Datenbank verschiedener Temperaturprofile ausgewählt oder individuell in Abhängigkeit von der größtmöglichen Kugel 14 bestimmt. Die Heizraten und Abkühlraten für ein Zahnersatzteil 1 mit einem Durchmesser 15 der größtmöglichen Kugel 14 oberhalb eines Grenzwertes von 6 mm dürfen beispielsweise einen Wert von 150 °C pro Minute nicht überschreiten. Denn dies würde zu thermischen Spannungen innerhalb des Zahnersatzteils 1 und damit zu Brüchen führen. Der Rohling 5 besteht aus einem Block 25 aus einem Sintermaterial 26 und der Rohlingshalterung 6.

Die Fig. 2 zeigt ein Temperaturprofil 30 für das Vorsintern des Rohlings 5 aus Fig. 1. Die Temperatur ist dabei in Abhängigkeit von der Zeit aufgetragen. In einer Aufheizphase 30 mit einer Heizrate 32 von beispielsweise 50 °C/ Stunde wird die Ofentemperatur bis zu einer Ansintertemperatur 33 von beispielsweise 1100 °C aufgeheizt. Anschließend wird in einer Haltephase 34 die Ansintertemperatur 33 für eine Haltezeit 35 von beispielsweise 60 Minuten gehalten. Danach erfolgt eine Abkühlphase 36 mit einer Abkühlrate 37 von beispielsweise 100 °C/Stunde. Durch das Vorsintern wird die notwendige Festigkeit des Rohlings 5 zur Bearbeitung in der CAM-Bearbeitungsmaschine erreicht. Eine zu hohe Ansintertemperatur 33 würde zu einer so hohen Festigkeit des Rohlings 5 führen, dass die Werkzeuge 9 der CAM-Bearbeitungsmaschine aus Fig. 1 schneller verschleißen würden.

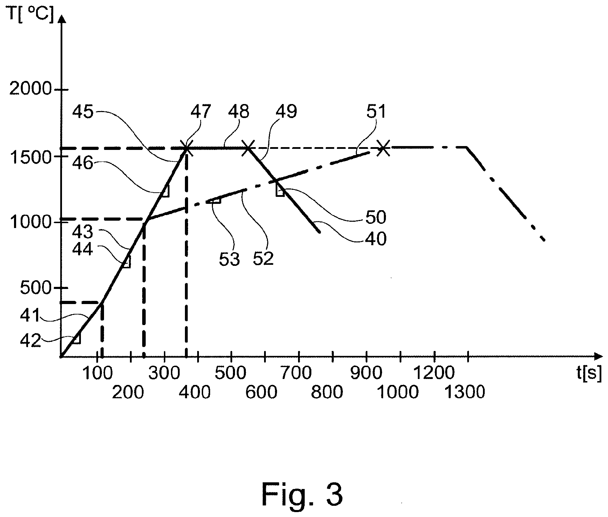

Die Fig. 3 zeigt ein Temperaturprofil 40 für das Dichtsintern des hergestellten Zahnersatzteils 1. In einer Trocknungsphase 41 wird die Ofentemperatur mit einer ersten Heizrate 42 in 120 Sekunden bis zu einer Temperatur von 400 °C erhöht. Die Dauer der Trocknungsphase kann bei einer höheren Festigkeit des hergestellten Zahnersatzteils 1 auch verkürzt werden. In einer ersten Aufheizphase 43 mit einer zweiten Heizrate 44 wird die Temperatur zwischen 120 und 250 Sekunden bis zu einer Temperatur von 1050 °C erhöht. In einer zweiten Aufheizphase 45 mit einer dritten Heizrate 46 von 265 °C pro Minute wird die Ofentemperatur in der Zeit zwischen 250 Sekunden und 370 Sekunden von einer Temperatur von 1050 °C bis zu einer Haltetemperatur 47 von 1580 °C erhöht. In einer Haltephase 48 wird die Haltetemperatur 47 für die Zeit zwischen 370 Sekunden und 550 Sekunden gehalten. In einer Abkühlphase 49 mit einer Abkühlrate 50 erfolgt dann die Abkühlung des Zahnersatzteils 1. Das Temperaturprofil 40 ist dabei für einen vorgesinterten Rohling 5 mit einer Ansintertemperatur 34 von 1100 °C dargestellt. Mit der Strichpunktlinie ist ein zweites Temperaturprofil 51 für ein Zahnersatzteil 1 aus einem vorgesinterten Rohling 5 dargestellt, der mit einer Ansintertemperatur 34 von 960 °C vorgesintert wurde. Die zweite Aufheizphase 52 des zweiten Temperaturprofils 51 weist eine deutlich geringere Heizrate 53 von 45 °C/Minute auf. Eine höhere Heizrate bei einem vorgesinterten Rohling mit der Ansintertemperatur von 960 °C würde wegen der geringeren Festigkeit zu mechanischen Spannungen und Brüchen führen. Der neuartige Rohling mit der höheren Ansintertemperatur ermöglicht also eine schnellere Aufheizphase 45 und verkürzt damit die Dauer der Dichtsinterung.

## Patentansprüche

1. Rohling (5) zur Herstellung eines Zahnersatzteils (1) unter Verwendung einer CAD/CAM-Vorrichtung (10, 7) aufweisend einen Block (25) aus einem Sintermaterial (26), wobei der Block (25) aus dem Sintermaterial (26) bei einer Ansintertemperatur (33) zwischen 1000 °C und 1250 °C in einen Sinterofen (2) vorgesintert ist, wobei das Sintermaterial (26) ein Pulver aus Keramikpartikeln mit einem Zirkoniumdioxid (Y-ZrO₂)- Gewichtsanteil von mindestens 90 % ist, wobei eine spezifische Oberfläche des Pulvers zwischen 11 m²/g und 17 m²/g beträgt, wobei das Sintermaterial (26) des Blocks (25) mindestens einen Farbstoff, eine Kombination mehrerer Farbstoffe und/oder mindestens ein Oxid oder ein Chlorid eines Farbstoffs zur Färbung des Blocks (25) enthält, wobei der mindestens eine Farbstoff Terbium für eine Gelbfärbung ist, der keinen Wertigkeitswechsel bei schnellem Abkühlen aufweist und damit keine Farbveränderung durchläuft.

2. Rohling (5) nach Anspruch 1, **dadurch gekennzeichnet, dass** die Ansintertemperatur (33) zwischen 1050 °C und 1200 °C beträgt.

3. Rohling (5) nach Anspruch 1 oder 2, **dadurch gekennzeichnet, dass** der Block (25) mit einem Pressdruck zwischen 130 und 250 MPa gepresst ist.

4. Rohling (5) nach einem der Ansprüche 1-3, **dadurch gekennzeichnet, dass** die spezifische Oberfläche des Pulvers zwischen 12 m²/g und 14 m²/g beträgt.

5. Rohling (5) nach einem der Ansprüche 1-4, **dadurch gekennzeichnet, dass** das Sintermaterial (26) des Blocks (25) einen Yttriumoxid- Gewichtsanteil zwischen 2 % und 4,5 % enthält.

6. Verfahren zur Herstellung eines Zahnersatzteils (1) aus einem Rohling (5) nach einem der Ansprüche 1 bis 5, wobei das herausgearbeitete Zahnersatzteil (1) in einem Sinterofen (2) nach einem festgelegten Temperaturprofil (40) dichtgesintert wird, wobei das Zahnersatzteil (1) eine größtmögliche Kugel (14) im Volumen des Zahnersatzteils (1) mit einem Durchmesser (15) unterhalb eines Grenzwertes von 6 mm aufweist, wobei das Temperaturprofil (40) in einer Aufheizphase (43, 45) eine festgelegte Heizrate (44, 46) zwischen 150 °C/Minute und 350 °C/Minute und/oder in einer Abkühlphase (49) eine festgelegte Abkühlrate (50) zwischen 150 °C/Minute und 350 °C/Minute aufweist.

7. Verfahren nach Anspruch 6, **dadurch gekennzeichnet, dass** das Zahnersatzteil (1) eine größtmögliche Kugel (14) im Volumen des Zahnersatzteils (1) mit einem Durchmesser (15) unterhalb eines Grenzwertes von 3 mm aufweist, wobei die festgelegte Heizrate (44, 46) zwischen 200 °C/Minute und 350 °C/Minute beträgt.

## Claims

1. A blank (5) for producing a tooth replacement part (1) using a CAD/CAM device (10, 7), comprising a block (25) made of a sintered material (26), the block (25) made of the sintered material (26) being pre-sintered at an initial sintering temperature (33) between 1000°C and 1250°C in a sintering furnace (2), the sintered material (26) being a powder made of ceramic particles with a zirconium dioxide (Y-ZrO₂) weight fraction of at least 90%, a specific surface area of the powder being between 11 m²/g and 17 m²/g, the sintered material (26) of the block (25) comprising at least one dye, a combination of several dyes and/or at least one oxide or a chloride of a dye for colouring the block (25), the at least one dye being terbium for a yellow colour, which has no change in valence with rapid cooling and thus does not undergo a change in colour.

2. The blank (5) according to claim 1, **characterised in that** the initial sintering temperature (33) is between 1050°C and 1200°C.

3. The blank (5) according to claim 1 or 2, **characterised in that** the block (25) is pressed with a pressure between 130 and 250 MPa.

4. The blank (5) according to one of claims 1-3, **characterised in that** the specific surface area of the powder is between 12 m²/g and 14 m²/g.

5. The blank (5) according to one of claims 1-4, **characterised in that** the sintered material (26) of the block (25) contains an yttrium oxide weight fraction of between 2% and 4.5%.

6. A method for producing a tooth replacement part (1) from a blank (5) according to one of claims 1 to 5, the machined tooth replacement part (1) being densely sintered in a sintering furnace (2) according to a defined temperature profile (40), the tooth replacement part (1) having a largest possible ball (14) in the volume of the tooth replacement part (1) with a diameter (15) below a limit value of 6 mm, the temperature profile (40) in a heating phase (43, 45) having a defined heating rate (44, 46) between 150°C/minute and 350°C/minute and/or in a cooling phase (49) having a defined cooling rate (50) between 150°C/minute and 350°C/minute.

7. The method according to claim 6, **characterised in that** the tooth replacement part (1) has a largest possible ball (14) in the volume of the tooth replacement part (1) with a diameter (15) below a limit value of 3 mm, the defined heating rate (44, 46) being between 200°C/minute and 350°C/minute.

## Revendications

1. Ébauche (5) pour la fabrication d'un élément prothétique dentaire (1) à l'aide d'un dispositif CAD/CAM (10, 7), comprenant un bloc (25) en un matériau fritté (26), le bloc (25) en le matériau fritté (26) étant préalablement fritté dans un four de frittage (2) à une température de frittage (33) comprise entre 1000 °C et 1250 °C, le matériau fritté (26) étant une poudre de particules de céramique présentant une fraction pondérale de dioxyde de zirconium (Y-ZrO₂) d'au moins 90 %, une aire spécifique de la poudre étant comprise entre 11 m²/g et 17 m²/g, le matériau fritté (26) du bloc (25) contenant au moins un colorant, une combinaison de plusieurs colorants et/ou au moins un oxyde ou un chlorure d'un colorant pour colorer le bloc (25), l'au moins un colorant étant du terbium pour une coloration en jaune, qui ne change pas de valence lors d'un refroidissement rapide et ne subit donc pas de changement de couleur.

2. Ébauche (5) selon la revendication 1, **caractérisée en ce que** la température de frittage (33) est comprise entre 1050 °C et 1200 °C.

3. Ébauche (5) selon la revendication 1 ou 2, **caractérisée en ce que** le bloc (25) est pressé avec une pression de compression comprise entre 130 et 250 MPa.

4. Ébauche (5) selon l'une des revendications 1-3, **caractérisée en ce que** l'aire spécifique de la poudre est comprise entre 12 m²/g et 14 m²/g.

5. Ébauche (5) selon l'une des revendications 1-4, **caractérisée en ce que** le matériau fritté (26) du bloc (25) contient une fraction pondérale d'oxyde d'yttrium comprise entre 2 % et 4,5 %.

6. Procédé de fabrication d'un élément prothétique dentaire (1) à partir d'une ébauche (5) selon l'une des revendications 1 à 5, l'élément prothétique dentaire (1) usiné étant fritté à densité maximale dans un four de frittage (2) selon un profil de température (40) déterminé, l'élément prothétique dentaire (1) présentant une bille (14) la plus grosse possible dans le volume de l'élément prothétique dentaire (1) avec un diamètre (15) inférieur à une valeur limite de 6 mm, le profil de température (40) présentant, dans une phase de chauffage (43, 45), une vitesse de chauffage déterminée (44, 46) comprise entre 150 °C/minute et 350 °C/minute et/ou, dans une phase de refroidissement (49), une vitesse de refroidissement déterminée (50) comprise entre 150° C/minute et 350° C/minute.

7. Procédé selon la revendication 6, **caractérisé en ce que** l'élément prothétique dentaire (1) présente une bille (14) la plus grosse possible dans le volume de l'élément prothétique dentaire (1) avec un diamètre (15) inférieur à une valeur limite de 3 mm, la vitesse de chauffage déterminée (44, 46) étant comprise entre 200 °C/minute et 350 °C/minute.
